# EUROPEAN PATENT SPECIFICATION

(11) **EP 0 332 912 B1**
(45) Date of publication and mention of the grant of the patent: **19.04.1995**
(21) Application number: 89103359.9
(22) Date of filing: 25.02.1989
(51) Int. Cl.: C07K 7/06, A61K 38/00, G01N 33/68

(54) **Inhibition of cell migration with synthetic peptides**
Hemmung von Migration von Zellen mit Hilfe von synthetischen Peptiden
Inhibition de la migration de cellules avec des peptides synthétiques

(30) Priority: 10.03.1988 US 166530
(43) Date of publication of application: 20.09.1989
(73) Proprietor: LA JOLLA CANCER RESEARCH FOUNDATION, La Jolla, CA 92037 (US)
(72) Inventor: Ruoslahti, Erkki I., Rancho Santa Fe, California 92067 (US); Pierschbacher, Michael D., San Diego, California 92107 (US); Gehlsen, Kurt R., San Diego, California 92122 (US)
(74) Representative: UEXKÜLL & STOLBERG

(56) References cited:
- EP-A- 0 275 748
- THE JOURNAL OF BIOLOGICAL CHEMISTRY, vol. 262, no. 36, 1987, pages 17294-17298,The American Society for Biochemistry and Molecular Biology, Inc.; M.D.PIERSCHBACHER et al.: "Influence of stereochemistry of the sequence Arg-Gly-Asp-Xaa on binding specificity in cell adhesion"
- CHEMICAL ABSTRACTS, vol. 108, 1988, page 391, abstract no. 219712r, Columbus,Ohio, US; K.R. GEHLSEN et al.: "Inhibition of in vitro tumor cell invasion by
- Arg-Gly-Asp-containing synthetic peptides",&& J. CELL. BIOL. 1988, 106(3), 925-30
- Hendrix, M. J. C. et al. (1985), Clin. Exp. Metastasis, 3(4), 221-223

## Description

The present invention relates to synthetic cell adhesion peptides and, more specifically, to their use in inhibiting cell migration.

The interactions of cells with extracellular matrix components such as fibronectin, vitronectin and type I collagen have been shown to be mediated through a family of cell surface receptors that specifically recognize an arginine-glycine-aspartic acid (Arg-Gly-Asp or RGD) amino acid sequence within each protein. Such receptor-ligand interactions are critical to the orderly migration and differentiation of cells and tissues during development, and to the continued maintenance of normal cell to cell interactions, and the binding specifity in cell adhesion is influenced by the stereochemistry of the sequence Arg-Gly-Asp-Xaa (Pierschbacher and Ruoslahti in J. Biol. Chem. 262, 1987, 17294-17298). Synthetic peptides containing the Arg-Gly-Asp sequence are capable of competing with adhesion proteins for their receptors, thereby inhibiting cell attachment of both normal and tumor cells on substrates coated with the adhesion proteins.

Occasionally, normal cell adhesion interactions may become disrupted resulting in inappropriate and potentially deleterious migration of cells, such as occurs in metastasis.

The metastatic process is comprised of a complex series of events, the details of which are largely unknown. For a tumor cell to be metastatic, it must be capable of attaching to the extracellular matrices that separate tissues and must penetrate such matrices. These matrices are composed of macromolecules that include fibronectin, laminin, collagens and proteoglycans. The interactions of cells with the extracellular matrix is mediated by cell surface receptors, including those which recognize and specifically bind to RGD containing sequences.

There thus exists a need for a composition capable of inhibiting the undesirable attachment to and/or penetration of extracellular matrices by cells. The present invention satisfies such a need and provides related advantages as well.

### Summary of the Invention

The present invention provides a method for inhibiting the invasion of cells, particularly malignant cells, through an extra-cellular membrane by contacting the membrane-cell interface with synthetic Arg-Gly-Asp-containing peptides. Preferably, a synthetic Arg-Gly-Asp-containing peptide is used for the preparation of a medicament for inhibiting cell invasion, wherein said synthetic Arg-Gly-Asp-containing peptide is R₁-Arg-Gly-Asp-X-R₂, wherein R₁ is one or more amino acids or H, X is Thr or other amino acid and R₂ is one or more amino acids or OH, whereby the total number of amino acids of said synthetic peptide does not exceed about 30, provided that neither R₁, X nor R₂ interfere with the ability of the peptide to inhibit cell invasion. In one embodiment, the invention provides peptides containing the amino acid sequence Arg-Gly-Asp-Thr, more specifically Gly-Arg-Gly-Asp-Thr-Pro, which inhibits the attachment of cells to type I collagen in addition to fibronectin and vitronectin, and a method of inhibiting the attachment of cells to type I collagen. Preferably, such peptides are stabilized to prevent their breakdown in a cellular environment. Such stabilization may be accomplished by conjugating the peptides to polymers such as polymeric sugars. The invention further provides an assay for quantitating the invasive quality of cells by determining the amount of such peptides necessary to prevent the cells from penetrating an extracellular membrane, such as an amniotic membrane, in vitro. In a preferred embodiment, the invention provides a method for quantitating the invasiveness of a cell line, comprising the steps of placing samples of cells from said cell line in each of several individual seeding chambers, each seeding chamber being separated from a corresponding target chamber by an extracellular membrane, incubating said samples of cells in said individual seeding chambers to allow attachment of said samples of cells to said extracellular membrane, adding increasing concentration of an RGD-containing peptide to said individual seeding chambers, incubating said samples of cells in the individual seeding chambers for a time sufficient to permit invasion of said samples of cells through the extracellular membrane, counting the number of cells which are present in each target chamber, and determining the concentration of RGD-containing peptide necessary to inhibit migration of said cells into the target chamber, said concentration providing a measure of the invasiveness of the cell line.

### Brief Description of the Drawings

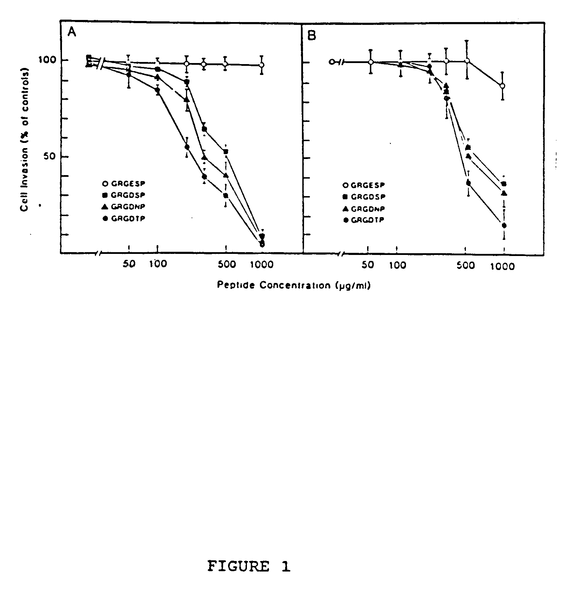

Figure 1 shows the effect of Arg-Gly-Asp-containing peptides on tumor cell invasion, using the MICS invasion system. The numbers of cells that had accumulated in the lower chamber compartment at 72 hours were used to calculate invasion. The uninhibited control values were set at 100% with the data derived from nine observations for each point. The mean and standard deviation is shown as a percentage of tumor cell invasion relative to the controls.

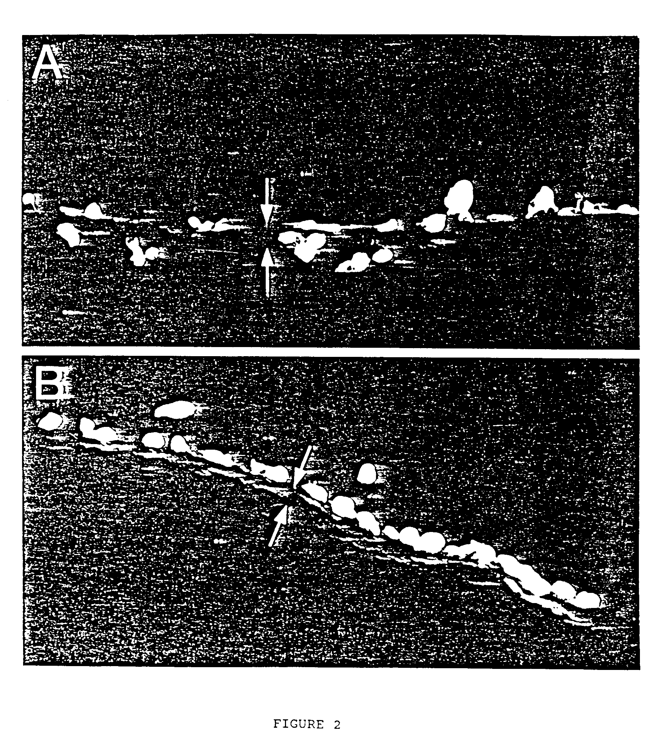

Figure 2 shows a visualization of invading tumor cells within the amniotic membrane. The cells were labelled with a fluorescent dye and allowed to invade the amniotic membrane for 72 hours in the presence of either Gly-Arg-Gly-Asp-Glu-Ser-Pro (GRGESP photograph A) or Gly-Arg-Gly-Asp-Thr-Pro (GRGDTP photograph B). The visualization of cell location during the invasion period was then determined by fluorescent microscopic examination of the sectioned amniotic membrane. Basement membrane surface (upper arrow) and the lower stromal surface (lower arrow) of the amniotic membrane are shown.

### Detailed Description of the Invention

The present invention relates to methods of inhibiting the invasion of cells through extracellular membranes by providing Arg-Gly-Asp-containing synthetic peptides. This inhibition is concentration dependent, non-toxic and correlates with both the ability of the peptides to interact with matrix adhesion receptors and with the invasive quality of the cells. Herein, all peptides are referred to by their standard three letter abbreviation, or one letter symbol, as detailed in, for example, U.S. Pat. No. 4,578,079, which is incorporated herein by reference.

For tumor cells to migrate through a membrane, such as an amniotic membrane, they must go through a series of steps. First, the cells have to attach to the basement membrane, a step which appears to be important in both in vitro as well as in in vivo invasion systems. Peptides containing the sequence Arg-Gly-Asp show only minimal inhibition of the attachment of tumor cells to the basement membrane and have no detaching effect after the cells have already attached to it. Next, the cells must penetrate into and through the stromal portion of the membrane, which in the amnion is about four-fifths the thickness of the membrane. While not wishing to be bound by this explanation, it is believed that the Arg-Gly-Asp containing peptides inhibit this step of the invasion process. In agreement with this belief is the fact that stromal extracellular matric contains the adhesive proteins fibronectin and type I collagen, cell attachment to which is specifically inhibited by the particular Arg-Gly-Asp-containing peptides shown to have the greatest inhibitory effect.

The invention further provides specific Arg-Gly-Asp-containing peptides having particular efficacy in inhibiting membrane invasion by tumor cells. Such peptides include those having the amino acid sequences Arg-Gly-Asp-Thr, and, more particularly, the sequence Gly-Arg-Gly-Asp-Thr-Pro. It is contemplated that such peptides may or may not include various other amino acids, or other chemical moieties, on either the amino terminal or carboxy terminal side of these specific sequences, provided that such additional moieties do not interfere with the ability of the peptides to inhibit membrane invasion, and, moreover, provided that such peptides are not identical to naturally occurring peptides.

The length of the synthetic peptides utilized in the present invention is limited by their solubility. Over about thirty amino acid residues the solubility becomes so reduced that they are not feasibly utilized. Preferably, the peptides under about thirty amino acids in length, more preferably four to ten, or more preferably five to eight.

Preferably, these peptides are stabilized so that they will not be quickly broken down or otherwise eliminated in a cellular or in vivo environment. Such stabilization may be effected by conjugating the peptides to organic polymers, such as polymeric sugars, preferably dextran. The molecular weight of the polymer can be specifically chosen to provide for the desired longevity, the higher molecular weight correlating with a longer half life of the conjugate.

There is further provided an in vitro assay system for quantitatively determining the invasive quality, or "invasiveness index" of particular cells. The assay system includes two chambers, the "seeding chamber" and the "target chamber" separated by an extracellular membrane, such as an amniotic membrane. Both chambers contain growth medium appropriate for the maintenance of the cells utilized. Cells suspected of being malignant are placed in the seeding chamber for a time sufficient to permit them to attach to and penetrate the membrane. Thereafter, the growth medium from one or both of the chambers is collected and the presence of cells therein determined. The presence of cells in the target chamber indicates that the cells are capable of membrane invasion.

In order to quantitate the degree of invasiveness possessed by the cells, the described assay is repeated with increasing concentrations of Arg-Gly-Asp-containing, invasion-inhibiting peptides being added to the seeding chamber. Such peptides may be added contemporaneously with the addition of the cells to the chamber, or preferably, may be added at a time determined to correlate with the penetration of the cells into and through the stromal portion of the membrane. The concentration of peptide necessary to achieve a particular level of invasion inhibition correlates with the invasiveness of the cells, providing an "invasiveness index".

Arg-Gly-Asp-containing peptides, particularly those stabilized so as not to be rapidly broken down in the body, may be useful in counteracting the invasion of tumor cells through connective tissue matrices. When provided with appropriate physiologically acceptable excipients and in amounts sufficient to inhibit such invasion, such Arg-Gly-Asp-containing peptides may be therapeutically useful in preventing the metastasis of tumor cells.

The following examples are intended to more clearly illustrate aspects of the invention, but are not intended to limit the scope thereof.

### EXAMPLE I

### Preparation of Synthetic Peptides

Peptides were synthesized using an automated peptide synthesizer (Model 430A; Applied Biosystems, Foster City, CA), using the instructions provided by the manufacturer, and purified by reverse phase HPLC on a Biogel TSK SP-5-PW cation exchange column (Bio-Rad Laboratories, Richmond, CA).

Where appropriate, cyclization was accomplished as follows. 611 mg of the synthesized peptide were dissolved in 4 L of water that had been previously boiled and allowed to cool. Immediately prior to addition of the peptide, nitrogen was bubbled through the water for 45 minutes. After the peptide was dissolved, a solution of 0.1 ug/mL of potassium ferrous cyanide K₃[Fe(CN)₆] in water was added dropwise to the stirred peptide solution until the yellow color persisted for 5 minutes (approximately 5 ml). The pH of the solution was held at 7.0 throughout this procedure by addition of NH₄OH. The solution was allowed to stand for 20 hours under low vacuum and then lyophilized. Excess K₃[Fe(CN)₆] was removed by passing the cyclized material over a Sephadex G-15 column (1.8 x 120 cm). The peptide was purified by reverse phase HPLC using a Waters Bondapak™ C₁₈ column (3 x 30 cm; 10 um packing) (Waters Assoc., Milford, MA). The peptide was loaded on the column in buffer A (20 mM ammonium acetate at pH 7.5) and eluted with a gradient of buffer B consisting of 60% acetonitrile and 40% buffer A. The major peak obtained from the C₁₈ column constituted 90% of recovered peptide and was deduced to be a monomeric cyclic peptide because it was retained on the column for the length of time predicted for that sequence and because the uncyclized material and the multimeric forms were well separated from the main peak.

The following peptides were synthesized for testing:

Gly-Arg-Gly-Glu-Ser-Pro
Gly-Arg-Gly-Asp-Ser-Pro
Gly-Arg-Gly-Asp-Asn-Pro
Gly-Arg-Gly-Asp-Thr-Pro
Gly-Arg-Gly-Asp-D-Ser-Pro
Gly-Pen-Gly-Arg-Gly-Asp-Ser-Pro-Cys-Ala
Gly-D-Arg-Gly-Asp-Ser-Pro

### Example II

### Cell Invasion Assays

Invasiveness of tumor cells was determined using a modification of the Membrane Invasion Culture System (MICS) as described in Hendrix et al. (1985) Clin. Exp. Metastasis 3:221-223. The cell lines used included two human melanoma cell lines designated A375P and A375M, the derivation and metastatic properties of which have been described in Kozlowski et al., (1984) J.N.C.L. 72:913, and a human glioblastoma cell line (RuGli) described in Goodman and Newgreen (1985) EMBO 4:2769. Noninvasive fibroblasts were used as control cells. All of the cells were cultured in DME (Gibco, Chagrin Falls, OH) supplemented with 10% heat inactivated fetal bovine serum (Tissue Culture Biologicals, Tulare, CA) and 0.1% gentamicin (Gibco). Cells were removed from culture dishes using 2 mM EDTA in PBS devoid of Ca⁺⁺ and Mg⁺⁺ for all assays. Cells (5 x 10⁴ to 1 x 10⁵) were radiolabelled with 0.25 uCi/ml ¹⁴C-thymidine (New England Nuclear (Boston, MA)) for 48 hours in DMEM containing 2% fetal bovine serum and then seeded into the upper compartment of the MICS chambers prepared as follows.

Fresh human placentae were obtained at birth, and in each case the amnion was obtained from near the region of the umbilical cord. After several rinses in sterile PBS, Fungi-zone/penicillin/streptomycin (Irvine Scientific, Irvine, CA) and PBS again, the amnion was trimmed to fit specially designed MICS chambers as depicted in Gehlsen and Hendrix, (1987) Pigment Cell 1:16-21, Fig. 1. The amnion was aspectically interposed between the top and bottom plates of the MICS apparatus, with the epithelial surface facing the top plate. The fastening screw was tightened and the extra membranous material trimmed away with a scalpel. Before fitting the membrane in the chamber, the bottom wells (the "target wells") were filled with sterile DMEM (Gibco) containing 10% fetal bovine serum. The amniotic epithelium was removed by treatment with freshly prepared 0.25 M ammonium hydroxide (NH₄OH) for 5 minutes at room temperature followed by extensive washing in PBS, which left a denuded basement membrane with an underlying collagenous stroma. In this manner, the interaction of cells with an extracellular matrix could be studied without the interference of host cells.

The labelled cells were delivered into each upper, or "seeding" chamber in serum-containing DMEM at a final concentration of 1.0 x 10⁵/ml and placed in a humidified incubator at 37 C with 5% CO₂ and 95% air atmosphere. All membranes were carefully examined for leakiness prior to cell seeding by allowing the denuded basement membrane surface to incubate for 1 hour with density marker beads (Pharmacia Fine Chemicals, Piscataway, NJ) with a buoyant density of 1.049 g/ml in a percoll gradient containing 0.25 M sucrose. If the colored beads, which were measured to be the same density as the cells from each cell line, were detected in the bottom wells of MICS, those portions of the membranes were not used.

The number of cells able to successfully invade the basement membrane and underlying collagenous stroma was determined by removing the media in the lower wells (1.1 ml) after each 24 hour increment and 72 hours via the side ports in the MICS chambers without disruption of the ongoing experiment. After each lower well was sampled, fresh media was replenished in the lower wells. In this manner, tumor cell invasion could be assessed repeatedly in the same experiment over various time intervals. The ¹⁴C-thymidine radio-labeled cells in all the lower well samples were pelleted and then placed in scintillation vials. In all experiments, the collected ¹⁴C-labelled cells were then lysed with 0.5 ml of 1 N NaOH. Aliquots of 100 ul glacial acetic acid were added to each vial to prevent chemiluminescence, and 10 ml of ACS scintillation cocktail (Amersham Corporation, Arlington Heights, IL) was added prior to radiolabel determination using a scintillation counter (Beckman Instruments).

For the peptide studies, peptides were added after a preattachment incubation period of 37 C in a 7% CO₂-air atmosphere and at 24 hour intervals thereafter, at which time medium in both the upper and lower compartments of the chambers was replaced with fresh, peptide-containing medium. At specific time intervals after the addition of peptides, medium from the upper and lower chambers was removed, the membranes were washed with fresh medium and the medium and wash fractions were centrifuged and the number of cells in the cell pellets was determined by counting the cell-associated radioactivity by liquid scintillation. The total number of cells that had passed through the membrane was derived by adding the lower chamber value to those from the previous samplings. In several experiments, unlabelled cells were used and the cells were counted in a hemocytometer. In addition, the number of cells that could partially but not completely invade the amnion was determined. The amnion was treated in tissue solubilizer (Amersham Corporation, Arlington Heights, IL) before counting cell-associated radioactivity.

Seven to nine percent of the A375M cells, 3 to 5% of the A375P cells and 7 to 12% of the RuGli cells seeded onto the membranes traversed the amnion in 72 hours. A marked decrease in the invasion was seen in the presence of Arg-Gly-Asp-containing peptides with all three cell lines. This inhibition of invasion was apparent at all time points analyzed, but it was greatest at 72 hours. For this reason, this time point was used in the subsequent experiments. Results from such an experiment in which several Arg-Gly-Asp-containing peptides and a control peptide were tested with the A375M and RuGli cells are shown in Fig. 1. The data shows that each of the Arg-Gly-Asp-containing peptides inhibited invasion, whereas the control peptide in which the aspartic acid has been substituted with glutamic acid rendering it inactive in cell attachment assays was without effect. A hexapeptide with a D-alanine in the place of the second glycine was also inactive.

The results shown in Fig. 1 indicate that Arg-Gly-Asp-containing adhesion proteins and their receptors play a role in tumor cell invasion. Differing degrees of inhibition were associated with specific sequences. The Gly-Arg-Gly-Asp-Thr-Pro peptide which, unlike Gly-Arg-Asp-Ser-Pro and Gly-Arg-Gly-Asp-Asn-Pro, inhibits the attachment of cells to type I collagen as well as to fibronectin and vitronectin was consistently the most active of those tested in the MICS system, suggesting that type I collagen plays some role in the invasion process. Gly-Arg-Gly-Asp-D-Ser-Pro, which inhibits attachment to fibronectin but not to vitronectin was as active as Gly-Arg-Gly-Asp-Ser-Pro, while the cyclic peptide, which inhibits attachment predominantly to vitronectin was an inefficient inhibitor of invasion.

### EXAMPLE III

### Visualization of Cell Location during Invasion

To visualize the invading cells in the amniotic membrane, fluorescently labelled cells were seeded onto amniotic membranes in MICS chambers as detailed in Example II. The labelled cells invaded as efficiently as unlabelled cells and the Gly-Arg-Gly-Asp-Thr-Pro peptide inhibited this invasion. Cross sections of the amniotic membranes were examined by fluorescent light microscopy to locate the cells. In experiments performed with a control peptide, Gly-Arg-Gly-Glu-Ser-Pro, cells at various stages of migration throughout the amnion were observed (Fig 2A), whereas examination of over 100 sections from experiments with the Gly-Arg-Gly-Asp-Thr-Pro peptide revealed no cells within the amnion stroma. In this case, the cells were located at the basement membrane surface or directly beneath the basement membrane (Fig. 2B).

Although the invention has been described with reference to the presently-preferred embodiments, it should be understood that various modifications can be made by those skilled in the art without departing from the invention. Accordingly, the invention is limited only by the following claims.

## Claims (Claims for the following Contracting State(s): AT, BE, CH, DE, FR, GB, IT, LI, LU, NL, SE)

1. Use of a synthetic Arg-Gly-Asp-containing peptide for the preparation of a medicament for inhibiting cell invasion wherein said synthetic Arg-Gly-Asp-containing peptide is R₁-Arg-Gly-Asp-X-R₂, wherein R₁ is one or more amino acids or H, X is Thr or other amino acid and R₂ is one or more amino acids or OH, whereby the total number of amino acids of said synthetic peptide does not exceed about 30, provided that neither R₁, X nor R₂ interfere with the ability of the peptide to inhibit cell invasion.

2. Use according to claim 1, wherein said peptide is Gly-Arg-Gly-Asp-Thr-Pro.

3. A method for quantitating the invasiveness of a cell line comprising the steps of:
a. placing samples of cells from said cell line in each of several individual seeding chambers, each seeding chamber being separated from a corresponding target chamber by an extracellular membrane;
b. incubating said samples of cells in said individual seeding chambers to allow attachment of said samples of cells to said extracellular membrane;
c. adding increasing concentrations of an RGD-containing peptide to said individual seeding chambers;
d. incubating said samples of cells in said individual seeding chambers for a time sufficient to permit invasion of said samples of cells through said extracellular membrane;
e. counting the number of cells which are present in each target chamber; and
f. determining the concentration of RGD-containing peptide necessary to inhibit migration of said cells into the target chamber, said concentration providing a measure of the invasiveness of the cell line.

## Claims (Claims for the following Contracting State(s): ES, GR)

1. A method for the production of a medicament for inhibiting cell invasion by synthesizing a peptide R₁-Arg-Gly-Asp-X-R₂, wherein R₁ is one or more amino acids or H, X is Thr or other amino acid and R₂ is one or more amino acids or OH, whereby the total number of amino acids of said synthetic peptide does not exceed about 30, provided that neither R₁, X nor R₂ interfere with the ability of the peptide to inhibit cell invasion.

2. The method of claim 1, wherein said peptide is Gly-Arg-Gly-Asp-Thr-Pro.

3. A method for quantitating the invasiveness of a cell line comprising the steps of:
a. placing samples of cells from said cell line in each of several individual seeding chambers, each seeding chamber being separated from a corresponding target chamber by an extracellular membrane;
b. incubating said samples of cells in said individual seeding chambers to allow attachment of said samples of cells to said extracellular membrane;
c. adding increasing concentrations of an RGD-containing peptide to said individual seeding chambers;
d. incubating said samples of cells in said individual seeding chambers for a time sufficient to permit invasion of said samples of cells through said extracellular membrane;
e. counting the number of cells which are present in each target chamber; and
f. determining the concentration of RGD-containing peptide necessary to inhibit migration of said cells into the target chamber, said concentration providing a measure of the invasiveness of the cell line.

## Patentansprüche (Patentansprüche für folgende(n) Vertragsstaat(en): AT, BE, CH, DE, FR, GB, IT, LI, LU, NL, SE)

1. Verwendung eines synthetischen Arg-Gly-Asp enthaltenden Peptids zur Herstellung eines Medikaments zur Hemmung der Zellinvasion, bei der das synthetische Arg-Gly-Asp enthaltende Peptid R₁-Arg-Gly-Asp-X-R₂ ist, wobei R₁ eine oder mehrere Aminosäuren oder H ist, X Thr oder eine andere Aminosäure ist, und R₂ eine oder mehrere Aminosäuren oder OH ist, und wobei die Gesamtanzahl von Aminosäuren des synthetischen Peptids etwa 30 nicht übersteigt, vorausgesetzt, daß weder R₁ noch X oder R₂ die Fähigkeit des Peptids zur Hemmung der Zellinvasion beeinträchtigen.

2. Verwendung nach Anspruch 1, bei der das Peptid Gly-Arg-Gly-Asp-Thr-Pro ist.

3. Verfahren zur Quantifizierung der Invasivität einer Zellinie, umfassend die Schritte:
a) Anordnung von Zellproben der Zellinie in jede von mehreren einzelnen Saatkammern, wobei jede Saatkammer von einer korrespondierenden Zielkammer mittels einer extrazellulären Membran getrennt ist;
b) Inkubieren der Zellproben in den einzelnen Saatkammern, um eine Anheftung der Zellproben an die extrazelluläre Membran zu erlauben;
c) Zugabe steigender Konzentrationen eines RGD enthaltenden Peptids zu den einzelnen Saatkammern;
d) Inkubieren der Zellproben in den einzelnen Saatkammern für eine Zeitdauer, die ausreicht, um eine Invasion der Zellproben durch die extrazelluläre Membran zu erlauben;
e) Ermittlung der Anzahl der Zellen die in jeder Zielkammer vorhanden sind; und
f) Bestimmung der zur Hemmung der Migration der Zellen in die Zielkammer erforderlichen Konzentration des RGD enthaltenden Peptids, wobei die Konzentration ein Maß für die Invasivität der Zellinie liefert.

## Patentansprüche (Patentansprüche für folgende(n) Vertragsstaat(en): ES, GR)

1. Verfahren zur Herstellung eines Medikaments zur Hemmung der Zellinvasion durch Synthese eines Peptids R₁-Arg-Gly-Asp-X-R₂, bei dem R₁ eine oder mehrere Aminosäuren oder H ist, X Thr oder eine andere Aminosäure ist, und R₂ eine oder mehrere Aminosäuren oder OH ist, wobei die Gesamtanzahl der Aminosäuren des synthetischen Peptids etwa 30 nicht übersteigt, vorausgesetzt, daß weder R₁ noch X oder R₂ die Fähigkeit des Peptids zur Hemmung der Zellinvasion beeinträchtigen.

2. Verfahren nach Anspruch 1, bei dem das Peptid Gly-Arg-Gly-Asp-Thr-Pro ist.

3. Verfahren zur Quantifizierung der Invasivität einer Zellinie, umfassend die Schritte:
a) Anordnung von Zellproben der Zellinie in jede von mehreren einzelnen Saatkammern, wobei jede Saatkammer von einer korrespondierenden Zielkammer mittels einer extrazellulären Membran getrennt ist;
b) Inkubieren der Zellproben in den einzelnen Saatkammern, um eine Anheftung der Zellproben an die extrazelluläre Membran zu erlauben;
c) Zugabe steigender Konzentrationen eines RGD enthaltenden Peptids zu den einzelnen Saatkammern;
d) Inkubieren der Zellproben in den einzelnen Saatkammern für eine Zeitdauer, die ausreicht, um eine Invasion der Zellproben durch die extrazelluläre Membran zu erlauben;
e) Ermittlung der Anzahl von Zellen, die in jeder Zielkammer vorhanden sind; und
f) Bestimmung der zur Hemmung der Migration der Zellen in die Zielkammer erforderlichen Konzentration des RGD-enthaltenden Peptids, wobei die Konzentration ein Maß für die Invasivität der Zellinie liefert.

## Revendications (Revendications pour l'(les) Etat(s) contractant(s) suivant(s): AT, BE, CH, DE, FR, GB, IT, LI, LU, NL, SE)

1. Utilisation d'un peptide synthétique contenant la séquence Arg-Gly-Asp pour la préparation d'un médicament destiné à inhiber l'invasion cellulaire dans lequel ledit peptide synthétique contenant la séquence Arg-Gly-Asp correspond à la formule R₁-Arg-Gly-Asp-X-R₂, où R₁ correspond à un ou plusieurs acides aminés ou à un atome d'hydrogène, X est un résidu Thr ou un autre acide aminé et R₂ correspond à un ou plusieurs acides aminés ou à un groupe OH, le nombre total d'acides aminés dudit peptide synthétique n'excédant pas 30 environ, sous réserve que ni R₁, ni X ni R₂ n'interfère avec l'aptitude du peptide à inhiber l'invasion cellulaire.

2. Utilisation selon la revendication 1 dans laquelle ledit peptide est Gly-Arg-Gly-Asp-Thr-Pro.

3. Procédé de mesure de l'aptitude d'une lignée cellulaire à l'invasion comprenant les étapes consistant à :
a. placer des échantillons cellulaires issus de ladite lignée dans plusieurs chambre d'ensemencement, chaque chambre d'ensemencement étant séparée d'une chambre cible correspondante par une membrane extracellulaire;
b. incuber lesdits échantillons cellulaires dans lesdites chambres d'ensemencement individuelles de manière à permettre l'attachement des échantillons cellulaires à ladite membrane extracellulaire;
c. ajouter des concentrations croissantes d'un peptide contenant la séquence RGD auxdites chambres d'incubation individuelles;
d. incuber lesdits échantillons cellulaires dans les chambre d'ensemencement individuelles pendant une durée suffisante pour permettre l'invasion de la membrane extracellulaire par lesdits échantillons cellulaires;
e. compter le nombre de cellules présentes dans chaque compartiment cible; et
f. déterminer la concentration de peptide contenant la séquence RGD nécessaire pour inhiber la migration desdites cellules dans la chambre cible, ladite concentration fournissant une mesure de l'invasion par la lignée cellulaire.

## Revendications (Revendications pour l'(les) Etat(s) contractant(s) suivant(s): ES, GR)

1. Procédé de préparation d'un médicament destiné à inhiber l'invasion cellulaire reposant sur la synthèse d'un peptide de formule R₁-Arg-Gly-Asp-X-R₂, où R₁ correspond à un ou plusieurs acides aminés ou à un atome d'hydrogène, X est un résidu Thr ou un autre acide aminé et R₂ correspond à un ou plusieurs acides aminés ou à un groupe OH, le nombre total d'acides aminés dudit peptide synthétique n'excédant pas 30 environ, sous réserve que ni R_{1,} ni X, ni R₂ n'interfère avec l'aptitude du peptide à inhiber l'invasion cellulaire.

2. Procédé selon la revendication 1 dans lequel ledit peptide est Gly-Arg-Gly-Asp-Thr-Pro.

3. Procédé de mesure de l'aptitude d'une lignée cellulaire à l'invasion comprenant les étapes consistant à :
a. placer des échantillons cellulaires issus de ladite lignée dans plusieurs chambre d'ensemencement, chaque chambre d'ensemencement étant séparée d'une chambre cible correspondante par une membrane extracellulaire;
b. incuber lesdits échantillons cellulaires dans lesdites chambres d'ensemencement individuelles de manière à permettre l'attachement des échantillons cellulaires à ladite membrane extracellulaire;
c. ajouter des concentrations croissantes d'un peptide contenant la séquence RGD auxdites chambres d'incubation individuelles;
d. incuber lesdits échantillons cellulaires dans les chambre d'ensemencement individuelles pendant une durée suffisante pour permettre l'invasion de la membrane extracellulaire par lesdits échantillons cellulaires;
e. compter le nombre de cellules présentes dans chaque compartiment cible; et
f. déterminer la concentration de peptide contenant la séquence RGD nécessaire pour inhiber la migration desdites cellules dans la chambre cible, ladite concentration fournissant une mesure de l'invasion par la lignée cellulaire.
